# EUROPÄISCHE PATENTSCHRIFT

(11) **EP 2 179 047 B1**
(45) Veröffentlichungstag und Bekanntmachung des Hinweises auf die Patenterteilung: **14.01.2015**
(21) Anmeldenummer: 08785279.4
(22) Anmeldetag: 01.08.2008
(51) Int. Cl.: C12P 7/64, C11C 1/00

(54) **Lipophile Zubereitungen**
Lipophilic preparations
Préparations lipophiles

(30) Priorität: 10.08.2007 DE 102007037783
(43) Veröffentlichungstag der Anmeldung: 28.04.2010
(73) Patentinhaber: Fabry, Bernd, 41352 Korschenbroich (DE); Gutsche, Bernhard, 40724 Hilden (DE)
(72) Erfinder: GUTSCHE, Bernhard, 40724 Hilden (DE); SCHÖRKEN, Ulrich, 40591 Düsseldorf (DE); WEISS, Albrecht, 40764 Langenfeld (DE); FABRY, Bernd, 41352 Korschenbroich (DE)
(74) Vertreter: Fabry, Bernd
(86) Internationale Anmeldenummer: PCT/EP2008/006338
(87) Internationale Veröffentlichungsnummer: WO 2009/021641

(56) Entgegenhaltungen:
- BOSWELL, K.D.B. ET AL.: "SCO Production by Fermentative Microalgae" INDUSTRIAL APPLICATIONS OF SINGLE CELL OILS, 1992, Seiten 274-286, XP001000911
- LÓPEZ-ELÍAS, J.A. ET AL.: "Mass production of microalgae in six commercial shrimp hatcheries of the Mexican northwest" AQUACULTURAL ENGINEERING, Bd. 29, Nr. 3-4, 2003, Seiten 155-164, XP002525083
- WEETE, J.D. ET AL.: "Lipids and Ultrastructure of Thraustochytrium sp. ATCC 26185" LIPIDS, Bd. 32, Nr. 8, 1997, Seiten 839-845, XP009077836

## Beschreibung

### Gebiet der Erfindung

Die Erfindung befindet sich auf dem Gebiet der oleochemischen Grundstoffe und betrifft neue Lipidzubereitungen, die auf der Grundlage spezieller Mikroorganismen gewonnen werden.

### Stand der Technik

Fettsäuren und deren Ester stellen wichtige Rohstoffe für eine Vielzahl von Industrien dar und finden als Vorprodukte vor allem für Waschmitteltenside, Schmierstoffe und kosmetische Inhaltsstoffe Verwendung. Die Natur liefert mit pflanzlichen Ölen und tierischen Fetten eine nachhaltige, ökologisch und ökonomisch wertvolle Rohstoffquelle, die mit der Vielzahl von zur Verfügung stehenden Fettsäurespektren eine große Zahl von industriellen Bedürfnissen abdeckt. Und doch ist der Stand der Technik weit davon entfernt, für alle bestehenden Probleme eine zufrieden stellende Lösung bereit zu halten.

Eine grundsätzliche Unzulänglichkeit bei der großtechnischen Herstellung von Fettsäuren und deren Derivaten durch Spaltung bzw. Umesterung von Fetten und Ölen besteht insbesondere darin, dass die Natur in verfügbaren Mengen nur solche Rohstoffe zur Verfügung stellt, die ein Überangebot an langkettigen gesättigten und ungesättigten Fettsäuren als auch an kurzkettigen Spezies enthalten, jedoch nur vergleichsweise geringe Mengen an Myristinsäure (C14) und Palmitinsäure (C16) enthalten. Diese Fettsäuren weisen aber gerade sowohl für kosmetische Anwendung als auch für den Einsatz in Waschmittel die optimale Kohlenstoffkettenlänge auf.

Neben diesen Rahmenbedingungen, die vom Markt vorgegeben werden, besteht das Erfordernis, die eingangs genannten Verfahren zur Herstellung von Fettsäuren und deren Alkylester als erste Produkte in der Wertschöpfungskette ökonomisch und ökologisch permanent zu verbessern. Abgesehen von den Rohstoffkosten tragen heute die Aufwendungen für Energie am stärksten zu den Herstellkosten bei. So führt beispielsweise schon das Verkürzen der Reaktionszeit um einige Minuten oder die Absenkung der Temperatur um wenige Grad Celsius gerade bei der Herstellung von Massenprodukten zu erheblichen Energie- und damit Kosteneinsparungen. Zu den technischen Problemen bei der Herstellung von Fettsäuren und deren Ester, die bislang einer Lösung harren, gehört die Abtrennung von Glycerin nach der Spaltung bzw. Umesterung, die unter ökonomisch akzeptablen Rahmenbedingungen immer noch eine zeitaufwendige Phasentrennung erforderlich macht.

In diesem Zusammenhang sei auf die Veröffentlichung von Boswell et al. "SCO Production by Fermentative Microalgae" in Ind. Applications of Single Cell oils, S. 274-286 (1992**)** hingewiesen, in er eine Fettsäurezusammensetzung mit der Bezeichnung EPASCO offenbart wird, die 21,9 Gew.-% Myristinsäure, 29,1 Gew.-% Palmitinsäure und 40,2 Gew.-% längerkettige C₁₈-C₂₀ Fettsäuren aufweist.

Die komplexe Aufgabe der vorliegenden Erfindung hat somit darin bestanden, lipophile Zubereitungen zur Verfügung zu stellen, die zum einen die Marktbedürfnisse treffen, also einen hohen Anteil an C₁₄- und C₁₆-Fettsäure(derivate)n aufweisen, und zum anderen den Vorteil einer gegenüber dem Stand der Technik vereinfachten technischen Herstellung besitzen.

### Beschreibung der Erfindung

Gegenstand der Erfindung sind lipophile Zubereitungen, enthaltend
(a) 20 bis 40 Gew.-% Myristinsäure oder deren Ester,
(b) 20 bis 40 Gew.-% Palmitinsäure oder deren Ester,
(c) 0,1 bis 5 Gew.-% aliphatische und/oder cycloaliphatische Kohlenwasserstoffe sowie
(d) weniger als 20 Gew.-% Carbonsäuren oder deren Ester mit 12 und weniger Kohlenstoffen im Acylrest und
mit der Maßgabe, dass sich alle Prozentangaben zu 100 Gew.-% ergänzen und wobei die aliphatischen und/oder cycloaliphatischen Kohlenwasserstoffe der Komponente (c) Squalene bzw. Squalane darstellen.

Die Mengenangaben zu (a) und (b) sind zudem so zu verstehen, dass die Gesamtmenge dieser beiden Komponenten 40 bis 80 Gew.-% ausmacht, wobei die einzelnen Spezies die oben angegebenen Grenzen auch gegebenenfalls unter- oder überschreiten können.

Überraschenderweise wurde gefunden, dass die erfindungsgemäßen Zubereitungen das eingangs erläuterte Anforderungsprofil in ausgezeichneter Weise erfüllen. Die Lipidfraktionen weisen zum einen den hohen Anteil an C₁₄ und C₁₆-Fettsäure(derivate)n auf, während die länger- und kürzerkettigen Spezies nur noch in geringen Mengen vertreten sind. Der Gehalt an Kohlenwasserstoffen führt dazu, dass die Abtrennung des Glycerins nach der Fettspaltung bzw. Umesterung um 10 % verkürzt werden kann, was zu einer signifikanten Steigerung der Anlagenkapazität sowie einer Verminderung des Energieverbrauchs führt. Weitere interessante Wertstoffe, wie beispielsweise Sterole oder Vitamine, sind in den Lipidfraktionen ebenfalls vorhanden und können nach Abtrennung und Aufreinigung die Rentabilität verbessern. Die wichtigste Erkenntnis, die dieser Erfindung zugrunde liegt, besteht indes darin, dass exakt solche Zubereitungen durch bestimmte Mikroorganismen, speziell Algen unmittelbar hergestellt werden. Besonders wichtig ist zudem, dass diese Mikroorganismen schon so hohe Lipidgehalte aufweisen, dass eine kommerzielle Nutzung als Rohstoffquelle möglich ist und eine Stammselektion oder genetische Modifikation lediglich zu einer Steigerung der Rentabilität des Verfahrens führt, ohne dafür die Voraussetzung zu bilden. Schließlich erlaubt die Erfindung den Einsatz von Abfallstoffen (Kohlendioxid aus Verbrennungsanlagen, Abwässer aus der Stärkeaufarbeitung) als Nährmedien für die Zellkulturen, was eine Kreislaufwirtschaft möglich macht, bei der schädliche Emissionen aus anderen Prozessanlagen reduziert.

Die Zubereitungen zeichnen sich des Weiteren dadurch aus, dass die Komponenten (a), (b), (d) und (e) unabhängig voneinander entweder als Voll- oder Partialglyceride oder als Ester mit aliphatischen Alkoholen mit 1 bis 4 Kohlenstoffatomen vorliegen.

### Herstellverfahren

Ein weiterer Gegenstand der Erfindung betrifft ein Verfahren zur Herstellung der oben genannten lipophilen Zubereitungen, bei dem man
(a) lipidproduzierende ein- oder mehrzellige Mikroorganismen, die
   (a1) einen Lipidgehalt - bezogen auf Trockenmasse -von wenigstens 10 Gew.-% aufweisen, wobei
   (a2) die Lipidfraktion
      (a21) 20 bis 40 Gew.-% Myristinsäure oder deren Ester,
      (a22) 20 bis 40 Gew.-% Palmitinsäure oder deren Ester,
      (a23) 0,1 bis 5 Gew.-% aliphatische und/oder cycloaliphatische Kohlenwasserstoffe vom Typ der Squalene bzw. Squalane sowie
      (a24) weniger als 20 Gew.-% Carbonsäuren oder deren Ester mit 12 und weniger Kohlenstoffen im Acylrest
      mit der Maßgabe enthält, dass sich alle Prozentangaben zu 100 Gew.-% ergänzen, kultiviert und
(b) die Mikroorganismen einer Extraktion unterwirft, bei der die Lipidfraktion von der Biomasse abgetrennt wird.

### Mikroorganismen

Bei den Mikroorganismen, die im Sinne der vorliegenden Erfindung, als Ausgangsstoffe für die Herstellung der C_{14/16}-Fettsäurereichen Lipidfraktionen, dienen, handelt es sich vorzugsweise um so genannte Mikro- oder µ-Algen. Dabei handelt es sich um eukaryotische, phototrophe, vorwiegend aquatische Mikroorganismen, die mit Hilfe von Chlorophyllen und Lichtenergie aus anorganischen Stoffen organische Stoffe erzeugen. Sie werden in die folgenden Klassen eingeteilt:
- *Cryptophyceae*
- *Dinophyceae*
- *Prymnesiophyceae*
- *Chrysophyceae*
- *Bacillariophyceae*
- *Dictyochophyceae*
- *Euglenophyceae*
- *Chlorophyceae*

Als typische Beispiele für besonders geeignete Mikroorganismen, insbesondere Mikroalgen und speziell Mikroalgen aus der Gattung Chrysophycea sind zu nennen:
- *Tetraselmis suecica,*
- *Nannochloropsis,*
- *Dinobryon divergens*
- *Mallomonas caudata*
- *Syncrypta globosa,*
- *Synura urella,*
- *Haptophycea isochrysis,*
- *Chaeto ceros,*
- *Paulova lutheri,*
- *Isochrysis galbana,*
- *Emiliana huxleyi* sowie
- *Prymnesiophycea parvum*
- *Isochrysis*
die bereits ohne Optimierung der Kultivierungsbedingungen oder Mutagenese bzw. gentechnische Verfahren Myristin/Palmitinsäuregehalte, von 30 bis 70 % aufweisen. Die oben genannten Vertreter sind aus dem Stand der Technik bekannt. So wird über deren Lipidzusammensetzung beispielsweise in von Mourente et al. [Hydrobiologia 203, 147 (1990**)],** Garrido et al. [J. Phycol. 36, 497 (2000**)],** Cobelas et al. [Grasas y Aceites 40, 118 (1989*)]* oder Elias et al. [Aquacultural Eng. 29,155 (2003**)]** berichtet ohne jedoch dabei auf Möglichkeiten einzugehen, wie gerade der gewünschte Gehalt an C_{14/16} Fettsäuren gesteigert werden kann oder welche Bedeutung der Gehalt an Kohlenwasserstoffen im Hinblick auf die prozesstechnischen Eigenschaften der Lipidfraktionen besitzen könnte. Alternativ kommen auch Diatomeen wie z. B. *Skeletonema costatum* oder *Phaeodactylum tricornutum* sowie Pilze wie z.B. Pythium in Frage.

### Kultivierung

Die optimale Kultivierung der Mikroorganismen stellt für die technische Lehre der vorliegenden Erfindung eine wichtige Randbedingung dar, da nur ein rasches Algenwachstum und hohe Lipidgehalte die Realisierung ökonomisch sinnvoll machen. Zwar ist es ohne weiteres möglich, beispielsweise die genannten Mikroalgen unter konventionellen Bedingungen zu züchten, in der Regel werden die dabei erhältlichen Lipidmengen bezogen auf die gesamte Trockenmasse jedoch zu gering ausfallen, um eine industrielle Nutzung - zumindest ohne geeignete Verfahren zur Aufkonzentrierung - attraktiv zu machen.

Aus dem Stand der Technik sind eine Vielzahl verschiedener Kultivierungsbedingungen und Nährmedien sowohl für kleine als auch industrielle Kulturen von Algenzellen bekannt. Im Rahmen der vorliegenden Erfindung hat es sich indes als besonders vorteilhaft erwiesen, von den folgenden Bedingungen auszugehen. Die Temperatur, bei der die Algenkulturen gezüchtet werden, gehört beispielsweise zu den besonders kritischen Parametern, da die Spezies aus unterschiedlichen Biotopen - Binnengewässern oder offenes Meer, warme oder kalte Regionen - stammen können und daher sehr unterschiedliche Vorlieben aufweisen können. Üblicherweise führt jedoch eine konstante Temperierung der Kulturen auf 20 bis 40 °C bei einem Optimum von etwa 30 °C zu besonders vorteilhaften Ergebnissen. Dabei wurde gefunden, dass durch eine höhere Temperatur in der Regel mit einem höheren Anteil an gesättigten Fettsäuren zu rechnen ist.

In einer weiteren bevorzugten Ausführungsform der vorliegenden Erfindung werden die Algen mixotroph gezüchtet, d.h. unter Zusatz zusätzlicher Nährstoffe für das Zellwachstum. Für diesen Zweck hat sich das so genannte "Arnon-Medium" als besonders vorteilhaft erwiesen, insbesondere wenn es mit Nitraten in Mengen von etwa 10 bis 60 mM und vorzugsweise von etwa 40 mM angereichert wird. Die Wachstumsrate kann ebenfalls durch die Zugabe von Salzen der Essigsäure, insbesondere Natriumacetat gesteigert werden, wobei die Zusatzmenge typischerweise im Bereich von 20 bis 60 mM und vorzugsweise etwa bei 40 mM liegt. Selbstverständlich ist es möglich, auch Mischungen von Nitraten und Acetaten einzusetzen. Ein weiterer Vorteil besteht allerdings auch darin, dass die genannten Mikroalgen auch die Verwendung von sehr preiswerten Nährmedien erlauben, beispielsweise Abwässern aus der Molkerei oder Stärkeaufarbeitung, die in großen Mengen praktisch kostenfrei zur Verfügung stehen und das erfindungsgemäße Verfahren dadurch zusätzlich attraktiv machen.

Die Bestrahlung der Algen stellt selbstverständlich ebenfalls eine kritische Größe dar. Dabei gilt es einerseits sicher zu stellen, dass die Algen eine ausreichende Menge Licht erhalten, ohne sich dabei andererseits zu stark zu beschatten. In der Pilotanlage hat sich eine Lichtmenge von 200 bis 1.500 µEm⁻²s⁻¹ und vorzugsweise 700 bis 1.000 µEm⁻²s⁻¹ als besonders vorteilhaft erwiesen. Im Produktionsmaßstab wäre es natürlich vorzuziehen, mit einer natürlichen Bestrahlung auszukommen.

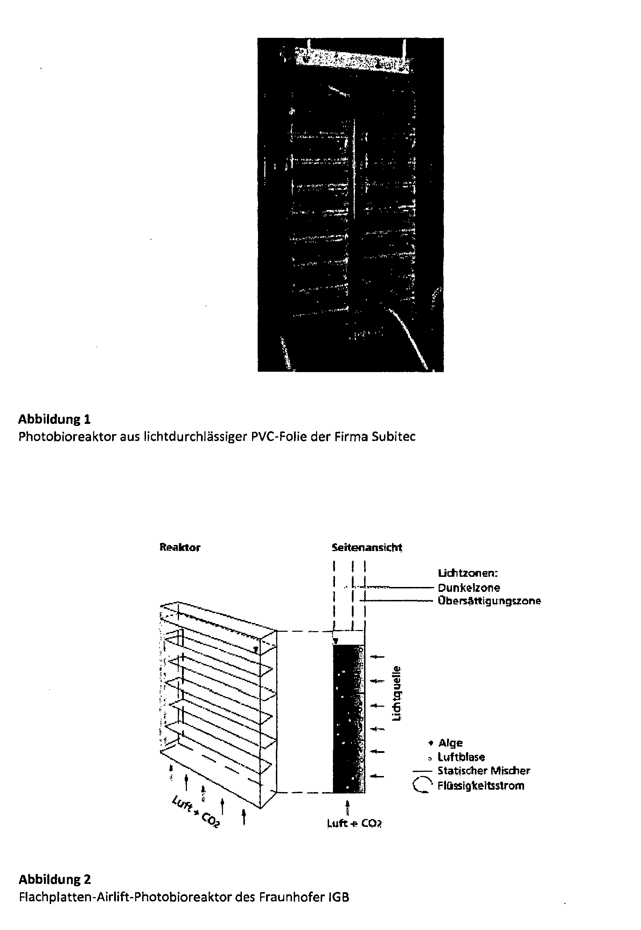

Ein besonderes Problem bei der Kultivierung von Algen besteht darin, dass mit steigender Konzentration an Algenmasse die eingestrahlte Menge Licht nur noch wenige Zentimeter in die Suspension eindringen kann, was dazu führt, dass untere Schichten praktisch nicht mehr bestrahlt werden. Um dies zu verhindern besteht eine weitere bevorzugte Ausführungsform der vorliegenden Erfindung darin, die Zellen in einem Photobioreaktor, vorzugsweise einem Röhren oder Flächenphotobioreaktor zu züchten. Diese Reaktoren weisen den besonderen Vorteil auf, dass sie bezogen auf ihr Volumen eine besonders große Oberfläche aufweisen, so dass die Kulturen jeweils nur dünne Schichten von etwa 4 bis 5 cm bilden und daher optimal bestrahlt werden. Das Volumen solcher Reaktoren kann je nach Produktionsmenge zwischen 100 und 50.000 I betragen, wobei als Materialien sowohl Glas als auch Kunststoffe, wie z.B. Polyacrylate, Polycarbonate oder PVC in Frage kommen. Entsprechende Vorrichtung sind aus dem Stand der Technik bekannt und werden beispielsweise von den Firmen iq-Brandenburg (Biostat BPR 3500), Subitec oder vom Fraunhofer IGB betrieben bzw. angeboten; zwei entsprechende Reaktoren ist beispielhaft in den Abbildungen 1 und 2 wiedergegeben.

Während der Wachstumsphase werden die Zellsuspensionen durch den Reaktor gepumpt, wobei das Wasser mit CO₂ angereichert wird. Dies dient insbesondere dem Zweck ein Verklumpen der Massen zu verhindern. Ein weiterer Vorteil im Hinblick auf die ausgewählten Mikroalgen besteht darin, dass diese ohne weiteres auch den Einsatz von ungereinigtem Kohlendioxid beispielsweise aus Verbrennungsanlagen zulassen. Hierdurch können schädliche Emissionen reduziert und umgekehrt sogar Emissionszertifikate erwirtschaftet werden.

Während der Kultivierung sollte die Zellkonzentration im Photobioreaktor vorzugsweise auf 0,1 bis 0,3 * 106 Zellen/ml bei einer Konzentration von 0,1 bis 0,4 g trockener Biomasse/I eingestellt werden, was beispielsweise durch gesteuerte Zugabe frischen Nährmediums erreicht werden kann. Wie schon oben erläutert, sollte die Lichtmenge 200 bis 1.500 µEm⁻²s⁻¹ und vorzugsweise 700 bis 1.000 µEm⁻²s⁻¹ betragen, wie sie beispielsweise von Quecksilberdampflampen geliefert wird. Im Photobioreaktor sollte die Temperatur zudem bei 20 bis 35 °C gehalten werden. Wie oben erläutert stellt die Temperatur gewissermaßen einen Schalter für den Sättigungsgrad der so erhaltenen Fettsäuregemische dar.

Wie eingangs erläutert sind die oben genannten Bedingungen für eine konventionelle Züchtung der Mikroalgen zwar vollauf geeignet, es kann jedoch erforderlich werden, die Bedingungen zu ändern, um die Algen zu einer vermehrten Produktion von Lipiden zu stimulieren, also die Ausbeuten zu verbessern und das Verfahren rentabler zu machen. Dies trifft insbesondere für die Fälle zu, in denen die Algen zwar hinsichtlich des Fettsäurespektrums schon optimiert sind, die Lipidmenge absolut betrachtet jedoch zu niedrig ist. Eine solche Stimulation kann durch Stressfaktoren erfolgen, die in den Algen einen Reflex zur vermehrten Bildung von Speicherstoffen auslösen. Zu diesem Zwecke kommen im Sinne des erfindungsgemäßen Verfahrens sehr unterschiedliche Faktoren in Betracht:
- Steigerung der Lichtintensität
- Entzug von Nährstoffen
- Chemischer bzw. oxidativer Stress sowie
- Änderung des pH-Wertes.

Überraschenderweise wurde gefunden, dass neben dem periodischen Zuführen und Verknappen von Nährstoffen im Gegensatz zur üblichen Fachmeinung gerade chemischer Stress zu einer vermehrten Synthese von Speicherlipiden führt. Hier hat sich insbesondere die Zugabe von peroxidischen Verbindungen, wie Wasserstoffperoxid in Mengen von 10 bis 50 mM als wirksam erwiesen

### Abtrennung und Aufarbeitung

Auch der Abtrennung und Aufarbeitung der Lipidfraktion von der restlichen Biomasse kommt eine große Bedeutung zu. Durch effiziente Trennverfahren ist es möglich, den Nachteil geringerer Lipidkonzentrationen wenigstens teilweise auszugleichen - wobei es natürlich auf der Hand liegt, dass die Kombination aus hohen Lipidgehalten und optimierten Aufarbeitung bevorzugt ist.

Die bevorzugte Methode besteht dabei im Sinne der Erfindung darin, die Suspensionen zu sedimentieren, zu filtrieren und/oder zu zentrifugieren, um die Algenmasse vom Nährmedium abzutrennen. Dazu hat es sich als sinnvoll erwiesen, den Suspensionen handelsübliche Flokkulierungsmittel zuzusetzen. Üblicherweise wird dazu nach jedem Kultivierungszyklus von 4 bis 6 Tagen die Biomasse aus dem Photobioreaktor abgelassen und über mehrere Stunden in einem Sedimentationsgefäß sich selbst überlassen, um die Abscheidung zu erleichtern. Das Verhältnis zwischen der noch feuchten Biomasse und der überstehenden wässrigen Nährmittellösung liegt dabei üblicherweise bei 30:70. Die wässrige Phase kann abgezogen und in den Kreislauf zurückgeführt werden, während der Rückstand vorzugsweise in einer Zentrifuge oder in einem Vakuumfilter getrocknet und aufkonzentriert wird. Die Menge an Trockenmasse beträgt dabei in Abhängigkeit vom gewählten Verfahren 30 bis 40 % bezogen auf die Ausgangsmasse.

Alternativ können die Lipidfraktionen den Algen auch durch einen "Melkprozess" entzogen werden. Hierzu werden die Algensuspensionen mit einem organischen Lösemittel behandelt und die Lipidfraktionen extrahiert. Dies bietet den Vorteil, dass die Algen wieder in den Photoreaktor zurückgeführt und zur weiteren Lipidsynthese genutzt werden können. Ein entsprechendes Verfahren ist Gegenstand der internationalen Patentanmeldung WO 03/095397 A2/A3(Cognis), deren Inhalt durch Bezugnahme hiermit eingeschlossen wird.

### Gewerbliche Anwendbarkeit

Gemäß der Lehre der vorliegenden Erfindung werden Lipidfraktionen gewonnen, die einen hohen Gehalt an C14 und C16-Fettsäuren aufweisen, während kürzer- und längerkettige Spezies in nur geringen Mengen vorhanden sind. Die Lipidfraktionen enthalten ferner Mengen an Kohlenwasserstoffen sowie zusätzlichen Wertstoffen, wie z.B. Sterole, Squalene und Vitamine. Nach der Konzentrierung können die Lipidfraktionen vom restlichen Wasser befreit werden, wozu sich insbesondere die Sprüh- oder Gefriertrocknung anbietet. Die auf diesem Wege erhältlichen Pulver können für verschiedene Einsatzzwecke sofort verwendet werden. In der Regel ist es jedoch wünschenswert, anstelle der Lipide die Fettsäuren oder deren Niedrigalkylester zu erhalten.

### Fettsäuregewinnung

Zur Gewinnung der Fettsäuren aus den Lipiden können die Konzentrate in an sich bekannter Weise entweder einer Veresterung oder einer Umesterung mit niederen Alkoholen unterworfen werden.

Eine Möglichkeit besteht darin, die Suspensionen einer thermischen, chemischen oder vorzugsweise enzymatischen Hydrolyse zu unterwerfen, wobei die freien Fettsäuren direkt aus den Zellmembranen in die flüssige Phase wandern und dort durch Sedimentation, Zentrifugation oder Filtration von der Biomasse abgetrennt werden können. Letztere kann verbrannt werden und liefert auf diese Weise dann Energie für das weitere Verfahren. Da es sich um einen nicht-fossilen Brennstoff handelt, besteht hier der weitere Vorteil, dass CO₂-Zertifikate nicht verbraucht, sondern umgekehrt erwirtschaftet werden, was das Verfahren sowohl ökologisch als auch ökonomisch interessant macht. Die Fettsäurefraktion kann dann mit niederen Alkoholen, vorzugsweise Methanol in an sich bekannter Weise verestert und anschließend fraktioniert bzw. aufgereinigt werden, um die gewünschten C-Schnitte zur Verfügung zu stellen.

In einer alternativen Ausführungsform der vorliegenden Erfindung können die Suspensionen auch ohne vorherige mechanische Abtrennung und Aufarbeitung direkt chemisch weiterverarbeitet werden. Hierzu bietet sich eine Umsetzung der Lipidfraktionen in der Suspension mit niederen Alkoholen an, wobei direkt die entsprechenden Alkyl-, vorzugsweise Methylester erhalten werden. Dies kann beispielsweise unter Druck in einem Doppelschraubenextruder erfolgen, wie dies für die Extraktion von Ölsaaten in der Europäischen Patentanmeldung EP 0967264 A1 *(Toulousaine de Recherche et Developpement AB)* beschrieben ist. Im Sinne der vorliegenden Erfindung werden hierzu vorzugsweise die Algensuspensionen mit dem niederen Alkohol - vorzugsweise Methanol - und einem handelsüblichen Umesterungskatalysator in einem Extruder gegeben und auf diese Weise ein mechanischer Aufschluss der Zellmembranen unter Freisetzung der Lipide erreicht. Anschließend wird die Mischung solange erhitzt, bis sich eine Glycerinphase bildet. Die partiell umgeesterte Mischung wird anschließend filtriert und beispielsweise in einem Rohrreaktor in an sich bekannter Weise der weiteren Umesterung unterworfen, bis ein nahezu vollständiger Umsatz erreicht ist. Die Reaktionsprodukte können anschließend falls gewünscht einer fraktionierten Destillation oder Rektifikation unterworfen und gegebenenfalls zu den Alkoholen hydriert werden.

### Beispiele

### Beispiel 1

### Fettsäuregewinnung ex Chaetoceros calcitrans/simplex

Herbizid-resistente Mutanten von 2 Mikro-Algen aus der Aquakultur nämlich Chaetoceros calcitrans und Chaetoceros simplex (Originalstämme verfügbar in der Northeast Pacific culture collection, British Columbia) wurden bei 25 °C in künstlichem Seewasser (Harrison et al. J. Phycol. (1980) 16:28-35) in einem 35 I-Rohrreaktor (Bauart QVF) mit einer durchschnittlichen Lichtintensität von 100 µE/m²s aufgezogen. Vorher fand der Scale-up der Inocculate in verschiedenen Stufen analog in gerührten Glasküvetten bzw. -fermentern statt, um den großen Reaktor betreiben zu können, Startkonzentration der Algenmasse 0,5 g/l. Der pH-Wert im System wurde auf 8,2 ± 0,2 durch Dosierung von Luft mit 2 % CO₂ konstant gehalten. Bei relativen Wachstumsraten von > 2 (Verdopplung der Zellmasse) und Lipidgehalten von > 50 % wurde nach 5 Tagen die Zellmasse geerntet und aufgearbeitet. Die Lipide wiesen die Zusammensetzungen (analysiert als Gesamtmethylester nach Ver-/Umesterung mit Methanol) gemäß **Tabelle 1** auf:

**Tabelle 1**

| Lipidzusammensetzungen [Gew.-%] | | |
|---|---|---|
| **Fettsäure-Methylestergehalt (bezogen auf 100 %)** | ***Chaetoceros calcitrans*** | ***Chaetoceros simplex*** |
| total C14 (ges. + unges.) | 28 | 35 |
| C16 | 54 | 53 |
| C18 | 3 | 4 |
| C20 | 12 | 6 |
| C22 | 1 | 1 |
| Ungerade FS (∑) C15/C17 | 2 | 2 |

### Beispiel 2

### Fettsäuregewinnung ex Isochyris sp.

Eine herbizid-resistente Mutante der Mikro-Alge *Isochrysis* sp. (T.ISO, CSRIO Algae Culture Collection) wurde über mehrere Stufen zu einem Feedmaterial von 0,5 g/l Reaktorvolumen für einen 30 I Rohrreaktor vom Typ (QVF) aufgezogen. Dabei wurde ein f2 Kultur-Medium (nach Guillardt Ryther, Gran. Can. J. Microbiol. (1962) 18: 229-39) verwendet. Der pH-Wert wurde durch kontrollierte Zufuhr eines Gemischs von Luft mit 5 % CO₂ auf einen pH-Wert von 8,5 ± 0,2 eingestellt. Die Fermentation findet bei einer Temperatur von 30 ± 2 °C statt. Das relative Zellwachstum lag bei 0,7 (Verdopplung der Zellmasse alle 3 Tage), nach 12 Tagen wurde die Biomasse geerntet, die Trockenmasse enthielt 35 % Lipidanteil, die Fettsäurezusammensetzung der extrahierten und in Methylester umgewandelten Fettsäurebestandteile lagen - unabhängig von der CO₂-Konzentration bei den Werten gemäß **Tabelle 2:**

**Tabelle 2**

| Lipidzusammensetzungen [Gew.-%] | |
|---|---|
| **Totalgehalte an Fettsäuremethylester** | **Isochrysis sp. (T.510)** |
| C14 | 29 |
| C16 | 41 |
| C18 | 19 |
| C20 | 1 |
| C22 | 9 |
| andere, ungerade Fettsäuren | 1 |

### Beispiel 3

### Einfluss des Squalangehaltes auf die Glycerinabscheidung

Die Algenmasse aus Beispiel 1 mit einem natürlichen Squalangehalt von 0,4 Gew.-% und einer Säurezahl von 4 wurde mechanisch von Wasser befreit und in einem Doppelschneckenextruder mit Methanol und Zinkacetat bei 180 °C Starttemperatur umgeestert. Dabei wurde ein Umesterungsgrad von 70 % der Theorie erreicht. Dazu wurden 30 Gew.-% Methanol sowie eine 1 Gew.-% Zinkacetat bezogen auf den Feedstrom von 5 kg /h zudosiert. Nach Abtrennung der Feststoffe durch Filtration erfolgte eine weitere Umsetzung in einem Rohrreaktor kombiniert mit jeweils einem Abscheider zur Glycerinabtrennung. Ein Rührreaktor von 5 I wurde bei 80 °C und einem Druck von 2 bar mit 1 kg des feststofffreien Produkts aus der Umsetzung im Extruder nach (destillativer) Wasserentfernung mit 30 Vol.%igem wässrigen Methanol und 0,3 % Vol.-%igem wässrigen Natriummethylat betrieben. Nach drei Stunden erfolgte ein Absetzen der Glycerinphase, nach Methanolentfernung und Wasserwäsche und Trocknung der Oberphase wurden 900 g Methylester mit einem Restgehalt an gebundenem Glycerin von 0,2 Gew.-%, entsprechend einem Umsatz von 99,8 % erhalten.

### Vergleichsbeispiel V1

Eine analoge Lipidzusammensetzung, die durch Abmischung entsprechender pflanzlicher Triglyceride hergestellt wurde und frei von Squalan war, wurde der gleichen Umesterung und Aufarbeitung wie gemäß Beispiel 3 unterworfen. Die Abscheidung der Glycerinphase erfolgte hierbei erst nach 3,5 h. Es wurden ebenfalls 900 g Methylester mit einem Restgehalt an gebundenem Glycerin von 0,4 Gew.-% erhalten.

### Beispiel 4

Die Algenmasse aus Beispiel 1 mit einem natürlichen Squalangehalt von 0,4 Gew.-% und einer Säurezahl von 1,5 wurde mechanisch von Wasser befreit und in einem Doppelschneckenextruder mit Methanol und Natriummethylat bei 80 °C Starttemperatur umgeestert. Dabei wurde ein Umesterungsgrad von 70 % der Theorie erreicht. Dazu wurden 30 Gew.-% Methanol sowie eine 0,3 Gew.-% Natriummethylat bezogen auf den Feedstrom von 5 kg /h zudosiert. Nach Abtrennung der Feststoffe durch Filtration erfolgte eine weitere Umsetzung. Ein Rührreaktor von 5 I wurde bei 80 °C und einem Druck von 2 bar mit 1 kg des feststofffreien Produkts aus der Umsetzung im Extruder nach (destillativer) Wasserentfernung mit 30 Vol.-%igem wässrigen Methanol und 0,3 % Vol.-%igem wässrigen Natriummethylat betrieben. Nach drei Stunden erfolgte ein Absetzen der Glycerinphase, nach Methanolentfernung und Wasserwäsche und Trocknung der Oberphase wurden 900 g Methylester mit einem Restgehalt an gebundenem Glycerin von 0,2 Gew.-%, entsprechend einem Umsatz von 99,8 % erhalten.

### Vergleichsbeispiel V2

Eine analoge Lipidzusammensetzung, die durch Abmischung entsprechender pflanzlicher Triglyceride hergestellt wurde und frei von Squalan war, wurde der gleichen Umesterung und Aufarbeitung wie gemäß Beispiel 3 unterworfen. Die Abscheidung der Glycerinphase erfolgte hierbei erst nach 3,5 h. Es wurden ebenfalls 900 g Methylester mit einem Restgehalt an gebundenem Glycerin von 0,4 Gew.-% erhalten.

## Patentansprüche

1. Lipophile Zubereitungen, enthaltend
(a) 20 bis 40 Gew.-% Myristinsäure oder deren Ester,
(b) 20 bis 40 Gew.-% Palmitinsäure oder deren Ester,
(c) 0,1 bis 5 Gew.-% aliphatische und/oder cycloaliphatische Kohlenwasserstoffe sowie
(d) weniger als 20 Gew.-% Carbonsäuren oder deren Ester mit 12 und weniger Kohlenstoffen im Acylrest und
mit der Maßgabe, dass sich alle Prozentangaben zu 100 Gew.-% ergänzen und wobei die aliphatischen und/oder cycloaliphatischen Kohlenwasserstoffe der Komponente (c) Squalene bzw. Squalane darstellen.

2. Zubereitungen nach Anspruch 1, **dadurch gekennzeichnet, dass** die Komponenten (a), (b), und (d) unabhängig voneinander als Voll- oder Partialglyceride vorliegen.

3. Zubereitungen nach Anspruch 1, **dadurch gekennzeichnet, dass** die Komponenten (a), (b), und (d) unabhängig voneinander als Ester mit aliphatischen Alkoholen mit 1 bis 4 Kohlenstoffatomen vorliegen.

4. Verfahren zur Herstellung von lipophilen Zubereitungen gemäß Anspruch 1, bei dem man
(a) lipidproduzierende ein- oder mehrzellige Mikroorganismen, die
(a1) einen Lipidgehalt - bezogen auf Trockenmasse - von wenigstens 10 Gew.-% aufweisen, wobei
(a2) die Lipidfraktion
(a21) 20 bis 40 Gew.-% Myristinsäure oder deren Ester,
(a22) 20 bis 40 Gew.-% Palmitinsäure oder deren Ester,
(a23) 0,1 bis 5 Gew.-% aliphatische und/oder cycloaliphatische Kohlenwasserstoffe vom Typ der Squalene bzw. Squalane sowie
(a24) weniger als 20 Gew.-% Carbonsäuren oder deren Ester mit 12 und weniger Kohlenstoffen im Acylrest
mit der Maßgabe enthält, dass sich alle Prozentangaben zu 100 Gew.-% ergänzen, kultiviert und
(b) die Mikroorganismen einer Extraktion unterwirft, bei der die Lipidfraktion von der Biomasse abgetrennt wird.

5. Verfahren nach Anspruch 4, **dadurch gekennzeichnet, dass** man als Mikroorganismen Mikroalgen einsetzt.

6. Verfahren nach Anspruch 5, **dadurch gekennzeichnet, dass** man Mikroalgen einsetzt, die ausgewählt sind aus der Gruppe, die gebildet wird von Tetraselmis suecica, Nannochloropsis, Dinobryon divergens, Mallomonas caudata, Syncrypta globosa, Synura urella, Haptophycea isochrysis, Chaetos ceros, Paulova lutheri, Isochrysis galbana, Emiliana huxleyi und Prymnesiophycea parvum sowie Stämmen, die aus ihnen durch Züchtung oder Mutagenese erhältlich sind.

7. Verfahren nach mindestens einem der Ansprüche 4 bis 6, **dadurch gekennzeichnet, dass** man die Mikroorganismen in einem Photobioreaktor kultiviert.

8. Verfahren nach mindestens einem der Ansprüche 4 bis 7, **dadurch gekennzeichnet, dass** man die Mikroorganismen bei Temperaturen im Bereich von 20 bis 40 °C kultiviert.

9. Verfahren nach mindestens einem der Ansprüche 4 bis 8, **dadurch gekennzeichnet, dass** man die Mikroorganismen mit Tageslicht oder einer künstlichen Lichtmenge von 200 bis 1.500 µEm⁻²s⁻¹ bestrahlt.

10. Verfahren nach mindestens einem der Ansprüche 4 bis 9, **dadurch gekennzeichnet, dass** man die Mikroorganismen mit Abwässern aus der Molkerei oder Stärkeaufarbeitung kultiviert.

11. Verfahren nach mindestens einem der Ansprüche 4 bis 10, **dadurch gekennzeichnet, dass** man die Mikroorganismen mit gereinigtem oder ungereinigtem Kohlendioxid aus Verbrennungsanlagen kultiviert.

12. Verfahren nach mindestens einem der Ansprüche 4 bis 11, **dadurch gekennzeichnet, dass** man die Mikroorganismen während der Wachstumsphase Stressfaktoren aussetzt.

13. Verfahren nach Anspruch 12, **dadurch gekennzeichnet, dass** man als Stressfaktoren die Steigerung der Lichtintensität, den Entzug von Nährstoffen, chemischen bzw. oxidativen Stress sowie eine Änderung des pH-Wertes einsetzt.

14. Verfahren nach mindestens einem der Ansprüche 4 bis 13, **dadurch gekennzeichnet, dass** man die Suspensionen der Mikroorganismen nach Beendigung der Wachstumsphase sedimentiert, filtriert und/oder zentrifugiert.

15. Verfahren nach mindestens einem der Ansprüche 4 bis 14, **dadurch gekennzeichnet, dass** man die Suspensionen der Mikroorganismen nach Beendigung der Wachstumsphase mit niederen Alkoholen umestert.

## Claims

1. Lipophilic compositions, comprising
(a) 20 to 40 % b.w. myristinic acid or their esters,
(b) 20 to 40 % b.w. palmitic acid or their esters;
(c) 0.1 to 5 % b.w. aliphatic and/or cycloaliphatic hydrocarbons, and
(d) less than 20 % b.w. carboxylic acids or their esters having 12 and less carbon atoms in its acyl residue,
on condition that the percentages add to 100 % whereby the aliphatic and/or cycloaliphatic hydrocarbons forming component (c) represent squalene and/or squalane.

2. The compositions of Claim 1, **characterized in that** components (a), (b) and (d) independently from each other represent full or partial glycerides.

3. The compositions of Claim 1, **characterized in that** components (a), (b) and (d) independently from each other represent esters with aliphatic alcohols having 1 to 4 carbon atoms.

4. A process for the production of lipophilic compositions of Claim 1, **characterized in that**
(a) lipid producing single or multi cellular micro-organisms,
(a1) showing a lipid content - calculated on dry mass - of at least 10 % b.w., whereby
(a2) said lipid fraction contains
(a21) 20 to 40 % b.w. myristinic acid or their esters,
(a22) 20 to 40 % b.w. palmitic acid or their esters;
(a23) 0.1 to 5 % b.w. aliphatic and/or cycloaliphatic hydrocarbons of squalene and/or squalane type, and
(a24) less than 20 % b.w. carboxylic acids or their esters having 12 and less carbon atoms in its acyl residue
on condition that the percentages add to 100 % b.w. are cultivated and
(b) said micro-organisms are subjected to extraction in order to separate said lipid fraction from biomass.

5. The process of Claim 4, **characterized in that** micro-algae are used as micro-organisms.

6. The process of Claim 5, **characterized in that** said micro-algae are selected from the group consisting of *Tetraselmis suecica, Nannochloropsis, Dinobryon divergens, Mallomonas caudata, Syncrypta globosa, Synura urella, Haptophycea isochrysis, Chaetos ceros, Paulova lutheri, Isochrysis galbana, Emiliana huxleyi et Prymnesiophycea parvum* and stems obtainable thereof by breeding or mutagenesis.

7. The process of at least one of Claims 4 to 6, **characterized in that** said micro-organisms are cultivated in a photo bioreactor.

8. The process of at least one of Claims 4 to 7, **characterized in that** said micro-organisms are cultivated at temperatures in the range of 20 to 40 °C.

9. The process of at least one of Claims 4 to 8, **characterized in that** said micro-organisms are irradiated with daylight or an artificial amount of light of from 200 to 1,500 µEm⁻²s⁻¹.

10. The process of at least one of Claims 4 to 9, **characterized in that** said micro-organisms are cultivated with sewage water from creameries or starch purification.

11. The process of at least one of Claims 4 to 10, **characterized in that** said micro-organisms are cultivated with purified or crude carbon dioxide from combustors.

12. The process of at least one of Claims 4 to 11, **characterized in that** during their growth period said micro-organisms are subjected to stress factors.

13. The process of Claim 12, **characterized in that** increase of light intensity, deprivation of nutrients, chemical or oxidative stress and change of pH value

14. The process of at least one of Claims 4 to 13, **characterized in that** after the end of the growth period the suspension of micro-organisms are subjected to sedimentation, filtration and/or centrifugation.

15. The process of at least on of Claims 4 to 14, **characterized in that** after the end of the growth period the suspension of micro-organisms is subjected to transesterification with lower alcohols.

## Revendications

1. Préparations lipophiles contenant
(a) 20 à 40 % (masse) acide myristique ou son ester
(b) 20 à 40 % acide palmitique ou son ester
(c) 0,1 à 5 % des hydrocarbures aliphatiques et/ou aromatiques
(d) moins de 20 % acides grasses ou ses esters avec 12 ou moins des atomes C dans le reste acylique
avec la condition que tous pourcentages (a) à (d) donnent dans l'addition 100% et les hydrocarbures (c) sont squalane et/ou squalene.

2. Préparations de la revendication 1, **caractérisé par** l'existence des components (a), (b) et (d) sous la forme des triglycérides ou glycérides partielles d'une manière indépendante.

3. Préparations de la revendication 1, **caractérisé par** l'existence des components (a), (b) et (d) sous la forme des esters des alcools aliphatiques avec 1 à 4 atomes de carbone d'une manière indépendante.

4. Procédé pour la fabrication des préparations lipophiles de la revendication 1 **caractérisé par** la culturation
(a) des micro-organismes uni- ou multicellulaires
(a1) contenant au minimum 10% de a matière lipidique (calculé sur la base de la biomasse sèche) avec
(a2) la fraction lipidique contient
(a21) 20 à 40% de l'acide myristique ou son ester
(a22) 20 à 40 % de l'acide palmitique ou son ester
(a23) 0,1 à 5 % des hydrocarbures aliphatiques et/ou aromatiques sous la forme de Squalane ou Squalene et
(a24) moins de 20 % acides grasses ou ses esters avec 12 ou moins des atomes C dans le reste de hydrocarbure acylique
avec la condition que tous pourcentages (a) à (d) donnent dans l'addition 100 %.
(b) et extraction de la biomasse pour séparer la fraction lipophilique de la biomasse.

5. Procédé suivant la revendication 4 **caractérisé par** l'utilisation des microalgues comme micro-organismes.

6. Procédé suivant la revendication 5 **caractérisé par** l'utilisation des microalgues de la groupe dont les membres sont Tetraselmis suecica, Nannochloropsis, Dinobryon divergens, Mallomonas caudata, Syncrypta globosa, Synura urella, Haptophycea isochrysis, Chaetos ceros, Paulova lutheri, *Isochrysis* galbana, Emiliana huxleyi et Prymnesiophycea parvum et les souches de ces microalgues produit par culture ou mutagénèse.

7. Procédé suivant au minimum une des revendications 4 à 6 **caractérisé par** l'utilisation d'un photo-bioréacteur pour la culturation.

8. Procédé suivant au minimum une des revendications 4 à 7 **caractérisé par** une culturation des microorganismes à des températures entre 20 et 40°C.

9. Procédé suivant au minimum une des revendications 4 à 8 **caractérisé par** l'irradiation des microorganismes par lumière du jour ou artificiel avec une intensité entre 200 et 1.500 µEm⁻²s⁻¹.

10. Procédé suivant au minimum une des revendications 4 à 9 **caractérisé par** l'utilisation des eaux usées de la laiterie ou production de l'amidon pour la culturation des microorganismes.

11. Procédé suivant au minimum une des revendications 4 à 10 **caractérisé par** l'utilisation du carbone dioxyde purifié ou brut venant d'une combustion dans la culturation.

12. Procédé suivant au minimum une des revendications 4 à 11 **caractérisé par** l'exposition des microorganismes pendant la culturation au stress.

13. Procédé suivant la revendication 12 **caractérisé par** l'utilisation des facteurs de stress comme augmentation de l'intensité lumineuse, privation des éléments nutritifs, changement du pH, stress chimique ou stress d'oxydation.

14. Procédé suivant au minimum une des revendications 4 à 13 **caractérisé par** le traitement de la suspension des microorganismes après la phase de grossissement par sédimentation, filtration et/ou centrifugation.

15. Procédé suivant au minimum une des revendications 4 à 14 **caractérisé par** la transesterification de la suspension des microorganismes après la phase de grossissement avec des alcools de chaine courte.
